# EUROPÄISCHE PATENTSCHRIFT

(11) **EP 2 172 159 B1**
(45) Veröffentlichungstag und Bekanntmachung des Hinweises auf die Patenterteilung: **28.12.2011**
(21) Anmeldenummer: 09171220.8
(22) Anmeldetag: 24.09.2009
(51) Int. Cl.: A61B 17/225

(54) **Stoßwellenkopf und Verfahren zu seiner Herstellung**
Shock wave head and method for its production
Tête d'onde de choc et son procédé de fabrication

(30) Priorität: 01.10.2008 DE 102008050151
(43) Veröffentlichungstag der Anmeldung: 07.04.2010
(73) Patentinhaber: Siemens Aktiengesellschaft, 80333 München (DE)
(72) Erfinder: Fehre, Jens, 91353 Hausen (DE); Nanke, Ralf Dr., 91077 Neunkirchen am Brand (DE); Opitz, Jörg Dr., 01219 Dresden (DE); Schreiber, Jürgen, Professor, 01189 Dresden (DE)

(56) Entgegenhaltungen:
- DE-A1- 4 227 800
- DE-A1- 10 225 709
- DE-A1- 19 500 673
- DE-A1-102005 059 271
- DE-A1-102005 060 734
- DE-A1-102007 030 285
- US-A1- 2009 099 484

## Beschreibung

Die Erfindung betrifft einen Stoßwellenkopf für eine Stoßwellenbehandlung wie z.B. die extrakorporale Stoßwellenlithotripsie (ESWL), bekannt beispielsweise aus DE 102 25 709 A1 oder DE 42 27 800 A1, sowie ein Verfahren zu seiner Herstellung. Ein Stoßwellenkopf für die genannte Anwendung umfasst zur Einleitung von ihm erzeugter akustischer Energie in den Körper eines Patienten einen Koppelbalg aus einem Polymermaterial wie Silikon oder PVC. Der Koppelbalg ist mit einem schallleitenden Ausbreitungsfluid wie Wasser gefüllt und durch seine balgartige Ausgestaltung verformbar. Bei der Durchführung einer Lithotripsie wird der Koppelbalg mit einem Ankoppelbereich an den Körper des Patienten gedrückt, wobei die Verformbarkeit des Balgs einen Ausgleich unterschiedlicher Wegstrecken zwischen einer Fokussierungseinheit des Stoßwellenkopfes, z.B. einer akustischen Linse, und dem Behandlungsziel, z.B. einem Nierenstein, gewährleistet. Neben einer guten Verformbarkeit muss der Koppelbalg passende akustische Eigenschaften, etwa eine geeignete akustische Impedanz aufweisen und röntgentransparent sein. Damit sind die Anforderungen an einen Koppelbalg bzw. an dessen Material noch nicht erschöpft. Das für die Herstellung eines Koppelbalgs verwendete Material muss leicht benetzbar sein. Zwischen dem Koppelbalg und der Haut des Patienten wird nämlich in aller Regel ein wasserhaltiges Gel als Koppelfluid gebracht, um einen problemlosen Übertritt der akustischen Wellen zu gewährleisten. Im Falle einer ungenügenden Benetzung der Balgoberfläche mit dem Koppelfluid bestünde die Gefahr, dass sich die Schallübertragung und die Bildqualität von innerhalb des Stoßwellenkopfes vorhandenen Schallortungssystemen verschlechtern. Je nach Herstellungsverfahren weisen Koppelbälge eine Oberflächenrauhigkeit im Bereich von z.B. Ra 0,1 bis 1 µm auf, was hinsichtlich einer guten Benetzbarkeit, der Anhaftung von Restschmutz und der Bildung von Kavitationskeimen nicht optimal ist. Gleiches gilt auch für die mit dem o.g. Ausbreitungsmedium in Kontakt stehende Innenseite des Koppelbalges. Neben den genannten Anforderungen muss ein Koppelbalg aus hygienischen Gründen leicht zu reinigen sein, was sich bei hydrophilen, also leicht mit wässrigen Fluiden benetzbaren Oberflächen schwieriger gestaltet bzw. in wünschenswerter Gründlichkeit nur mit erhöhtem Einsatz aggressiver Reinigungschemikalien möglich ist. Aus hygienischen Gründen wäre daher ein Koppelbalg mit einer hydrophoben Oberfläche günstig, wobei sie dann aber nicht mehr mit Koppel- oder Ausbreitungsfluid benetzbar wären.

Die DE 102 25 709 A1 bildet die Basis für den Oberbegriff der Ansprüche 1 und 11. Neben dem weiter oben genannten Stand der Technik ist noch die Druckschrift DE 10 2005 059 271 zu erwähnen, in der eine Kathetervorrichtung mit einer Beschichtung aus Silikonmaterial und Nanopartikeln beschrieben ist.

Davon ausgehend ist es die Aufgabe der Erfindung, einen Stoßwellenkopf vorzuschlagen, der hier Abhilfe schafft.

Diese Aufgabe wird durch einen Stoßwellenkopf mit den Merkmalen des Anspruchs 1 sowie ein Verfahren nach Anspruch 11 gelöst. Ein erfindungsgemäßer Stoßwellenkopf zeichnet sich dadurch aus, dass außenseitig am Ankoppelbereich des Koppelbalges polare funktionelle Gruppen tragende Nanopartikel vorhanden sind. Unter einer polaren funktionellen Gruppe ist ein Molekül zu verstehen, das zumindest einen Teil einer Elementarladung (+e oder -e) trägt. Derartige Nanopartikel ermöglichen eine einfach durchzuführende und fest haftende Beschichtung der Balgoberfläche, indem nämlich die polaren Gruppen und damit das sie tragenden Nanopartikel aufgrund von elektrostatischen Wechselwirkungen mit polaren Atomen oder Atomgruppen des polymeren Werkstoffs (was bei den üblicherweise für Koppelbälge verwendeten Polymeren der Fall ist) an der Balgoberfläche festgehalten werden, wobei noch Van-der-Waals Kräfte und evtl. Wasserstoffbrücken hinzukommen. Eine Oberfläche der genannten Art lässt sich nicht nur mit geringem Aufwand herstellen, etwa einfach durch Aufbringen einer Nanopartikel-Suspension auf die Balgoberfläche und einer sich anschließenden Trocknung. Sie lässt sich auch hinsichtlich ihrer Benetzbarkeit verändern. Beispielsweise kann dies dadurch bewerkstelligt werden, dass ein mit der Balgoberfläche in Kontakt zu bringendes Fluid einen Elektrolyten, also einen in wässriger Lösung in Form von Ionen vorliegenden Stoff enthält. Je nach Konzentration des Elektrolyten lassen sich die Ladungsverhältnisse der mit den Nanopartikeln versehenen Oberfläche und damit deren Benetzungseigenschaften verändern.

Bei einer bevorzugten Ausführungsvariante werden Nanopartikel mit Säure- und/oder Basengruppen eingesetzt. In einem solchen Fall lässt sich die Ladung der mit Nanopartikeln besetzten Oberfläche des Koppelbalgs durch den pH-Wert des mit ihr in Kontakt gebrachten Fluids beeinflussen. In wässriger Lösung dissoziieren die Säuregruppen unter Abspaltung wenigstens eines Protons, wobei sich ein Dissoziationsgleichgewicht einstellt und zumindest an einem Teil der funktionellen Gruppen ein negativ geladener Säurerest verbleibt. Die mit den Nanopartikeln beschichtete Oberfläche wird dabei hydrophil, wird also mit einem wässrigen Fluid benetzt. Der pH-Wert des Fluids muss dabei so eingestellt sein, dass die Dissoziation der Säuregruppe nicht behindert wird, was dann der Fall wäre, wenn das Fluid eine hohe Konzentration an Protonen bzw. Oxoniumionen aufweisen würde. Dann würde nämlich entsprechend dem Massenwirkungsgesetz das Dissoziationsgleichgewicht auf die Seite der undissoziierten Säuregruppe verschoben werden. Es ist daher z.B. ein Fluid zu verwenden, das neutral oder basisch ist. Während der Durchführung einer ESWL kann der Koppelbalg somit allein durch den pH-Wert etwa eines Koppelfluids hydrophil eingestellt werden und dadurch optimale schallakustische Bedingungen geschaffen werden, wobei die Bildung von Lufteinschlüssen und Kavitationen verhindert ist.

Zur Reinigung des Koppelbalgs nach einer ESWL kann die Oberfläche des Koppelbalgs in einen die Reinigung erleichternden hydrophoben Zustand gebracht werden, indem eine saure Reinigungsflüssigkeit, also eine solche mit erhöhter Oxoniumionenkonzentration verwendet wird. Dabei wird, wie weiter oben schon geschildert das Dissoziationsgleichgewicht auf die Seite der undissoziierten Säuregruppe verschoben, wodurch die Oberflächenladung verringert und dementsprechend die Oberfläche hydrophob wird. Das bedeutet, dass die adhäsiven Anziehungskräfte zwischen der Balgoberfläche und den Wassermolekülen wesentlich geringer als zwischen den Wassermolekülen selbst, so dass die Oberfläche praktisch nicht mehr benetzbar ist. Eine derart eingestellte Balgoberfläche lässt sich leicht reinigen, wobei nur ein geringer Einsatz von Reinigungschemikalien erforderlich ist.

Im Falle einer Basengruppe als funtionelle Gruppe nimmt diese wenigstens ein Proton aus dem Wasser eines mit der Balgoberfläche in Kontakt gebrachten Koppelfluids (oder auch eines Ausbreitungsfluids) auf. Im Ergebnis bildet sich eine mehr oder weniger stark positiv geladene - hydrophile - Oberfläche. Während das Koppelfluid einen relativ geringen pH-Wert aufweisen sollte, wird bei zur Reinigung des Koppelbalges eine Reinigungslösung verwendet, die möglichst wenige Protonen bzw. Oxoniumionen enthält, die also einen hohen pH-Wert aufweist, so dass sich das Dissoziationsgleichgewicht auf die Seite der freien (ungeladenen) Basengruppe verschiebt. Die Balgoberfläche befindet sich dann im hydrophoben Zustand.

Werden schließlich Nanopartikel verwendet, an die sowohl saure als auch basische funktionelle Gruppen oder Gruppen mit einer sauren und einer basischen Funktion gebunden sind, so liegen vergleichbare Verhältnisse vor. In diesen Fällen lässt sich durch geeignet Wahl des pH-Werts des mit der Oberfläche in Kontakt gebrachten Fluids, ein Zustand mit einer neutralen Summenladung einstellen. An diesem Punkt, dem isoelektrischen Punkt, ist die Balgoberfläche maximal hydrophob eingestellt. Mit einem Fluid, das vom pH-Wert des isoelektrischen Punkts in saurer oder basischer Richtung abweicht, können hydrophile Verhältnisse eingestellt werden.

Bei einer weiteren bevorzugten Ausgestaltung werden Nanodiamanten als Partikel eingesetzt. Nanodiamanten weisen eine Größe etwa im Bereich von 50 nm auf und sind am Markt erhältlich (z.B. Firma Plasmachem, Berlin). Sie lassen sich beispielsweise durch kontrollierte Detonation eines mit Kohlenstoff versetzten Sprengstoffs wie TNT herstellen. Nanodiamanten haben u.a. den Vorteil, dass sich ihre Kohlenstoffatome gut zur kovalenten Anbindung organischer Gruppen oder zur reaktiven Bildung solcher Gruppen eignen. So können durch eine Oxidation mit Hilfe eines Gemisches aus Schwefel- und Salpetersäure Nanodiamanten mit Carboxylgruppen erhalten werden (Huang, L.-C. L.: Adsorption and Immobilization of Cytochrome c on Nanodiamonds. In: Langmuir (2004) H. 20, S. 5879-5884; Yang, J.-H. et al.: Functionalization of ultradispersed diamond for DNA detection. In: Springer Research Paper (2008). Derartige Nanodiamanten sind besonders bevorzugt, weil die Carboxylgruppen an polaren Polymeren mit einer ausreichender Festigkeit haften und das Umschalten von einem hydrophilen in einen hydrophoben Zustand der Oberfläche in einem pH-Wertbereich von leicht sauer bis schwach basisch erfolgen kann. Fluide mit einem pH-Wert innerhalb des genannten Bereichs sind schonend für den Koppelbalg und sonstige Teile des Stoßwellenkopfes und sind aufgrund ihrer relativ geringen Aggressivität leicht zu handhaben. Neben Nanodiamanten ist es auch denkbar, Nanokohlenstoff einzusetzen.

Wie bereits erwähnt, ist es zur Erzeugung einer fest auf der Balgoberfläche haftenden Beschichtung mit Nanopartikeln vorteilhaft, Koppelbälge aus polaren Polymeren wie Silicon oder PVC einzusetzen. Daneben sind aber auch andere Kunststoffe wie PA6, POM, PBT, PEEK, EP, PF, MF, PUR und UP denkbar.

Bisher wurde davon gesprochen, dass die auf der Oberfläche des Koppelbalgs vorhanden Nanopartikel nachträglich auf die Balgoberfläche aufgebracht werden. Es ist aber auch denkbar, dass funktionellen Gruppen tragende Partikel im Balgmaterial enthalten sind oder dass der Koppelbalg mehrschichtig aufgebaut ist, wobei in einer äußeren Materialschicht Nanopartikel vorhanden sind.

Nanopartikel der in Rede stehenden Art können nicht nur auβenseitig am Ankoppelbereich vorhanden sein, sondern auf der gesamten Außenseite des Koppelbalgs, z.B. um die Reinigung des gesamten Koppelbalgs auf einfache Weise zu ermöglichen. Denkbar ist auch, dass nicht nur der Ankoppelbereich innenseitig eine gute Benetzbarkeit bewirkende Nanopartikel aufweist, sondern die gesamte Innenseite solche Partikel trägt, um Lufteinschlüsse und Kavitationen zu vermeiden.

Zur Herstellung eines erfindungsgemäßen Stoßwellenkopfes wird eine Beschichtung des Koppelbalgs vorgenommen, indem auf die Außen- und/oder Innenseite des Koppelbalges eine wässrige Suspension der Nanopartikel aufgebracht und anschließend der Koppelbalg einer Trocknung unterzogen wird. Dieses Verfahren ist einfach durchzuführen, erfordert insbesondere einen geringen gerätetechnischen Aufwand, vor allem wenn der Koppelbalg in die Suspension eingetaucht wird (dip-coating).

Wie bereits erwähnt wurde, werden die Nanopartikel fest an die Balgoberfläche gebunden, wenn zwischen den polaren Atomen oder Atomgruppen des Balgmaterials und den Nanopartikeln elektrostatische Anziehungskräfte wirken. Dies ist dann in hohem Ausmaß gewährleistet, wenn sich die funktionellen Gruppen in einem Zustand möglichst hoher Ladung befinden. Im Falle von sauren-und/oder basischen Gruppen wird dies bei einer besonders bevorzugten Verfahrensvariante dadurch erreicht, dass in der Partikelsuspension, etwa durch Zugabe eines Säure-Base-Puffers, ein pH-Wert eingestellt wird, bei die Säure-Base-Reaktion zwischen den Gruppen und dem Wasser der Suspension zumindest teilweise stattfindet. Im Falle einer sauren Gruppe ist ein hoher pH-Wert zu wählen, so dass diese durch Abgabe eines Protons an das Wasser möglichst weit dissoziert ist und dementsprechend negativ geladen ist. Bei einer basischen Gruppe ist dagegen der pH-Wert so einzustellen, dass möglichst viele Gruppen mit dem Wasser der Suspension unter Anlagerung eines Protons reagieren und sich dadurch positiv aufladen.

Bei einer bevorzugten Verfahrensvariante werden Nanopartikel, insbesondere Nanodiamanten mit Carboxygruppen eingesetzt, wobei hier ab einem pH-Wert der Suspension von pH = 6 eine gute Haftung der Nanopartikel an der Oberfläche eines Koppelbalgs, insbesondere eines solchen aus Silicon, erreicht wird. Die jeweils verwendeten Suspensionen weisen Partikelgehalte von 0,1 g/l bis 1,5 g/l auf, um eine ausreichende Belegung der Balgoberfläche zu gewährleisten. Vorzugsweise wird in einem Bereich von 0,3 g/l bis 0,7 g/l gearbeitet.

Die Erfindung wird nun unter Bezugnahme auf die beigefügten Abbildungen näher erläutert. Es zeigen:

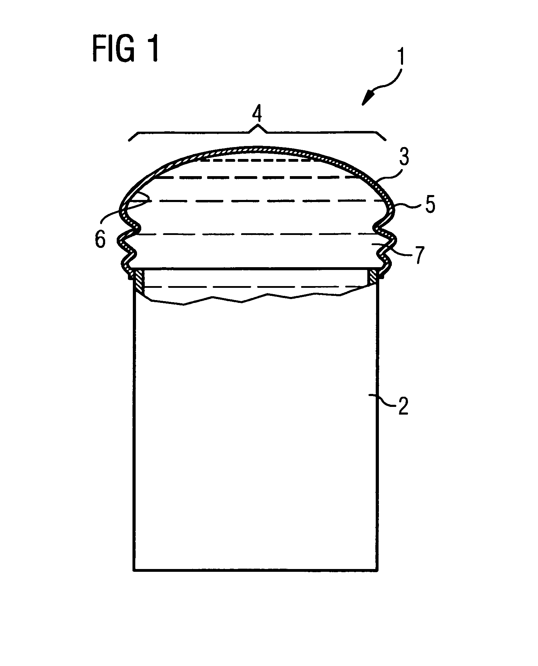

- Fig. 1: eine schematische Darstellung eines Stoßwellenkopfes,
- Fig. 2: ein Diagramm, welches die Abhängigkeit mit Nanodiaman- ten beschichteter Koppelbalgoberflächen von dem pH- Wert einer auf die Oberfläche aufgebrachten Lösung zeigt,
- Fig. 3: eine schematische Darstellung, welche die Bestimmung des Kontaktwinkels eines auf eine Oberfläche aufge- brachte Flüssigkeitstropfens aufzeigt.

Der Stoßwellenkopf 1 einer ESWL-Vorrichtung umfasst ein etwa topfförmiges Gehäuse 2, an dessen offener Seite ein Koppelbalg 3 fixiert ist. Der Koppelbalg 3 ist mit einem Ausbreitungsfluid 7, in der Regel Wasser oder eine wässrige Lösung, gefüllt. Im Gehäuse befindet sich u.a. eine Stoßwellenquelle und gegebenenfalls eine akustische Linse (beides nicht gezeigt). Der Koppelbalg 3 wird bei der Durchführung einer Lithotripsie an den Körper eines Patienten gepresst, wobei sich ein Bereich des Koppelbalges 3, dessen Ankoppelbereich 4 abflacht, bzw. der Kontur des Patientenkörpers anpasst. Zur Übertragung des von dem Stoßwellenkopf erzeugten Schalls in dem Patientenkörper wird zwischen den Koppelbalg 3 bzw. dem Ankoppelbereicht 4 und dem Körper des Patienten ein Koppelfluid (nicht gezeigt) gebracht. Das Koppelfluid ist in der Regel ein gelartiges wasserhaltiges Fluid. Der Koppelbalg 3 eines Stoßwellenkopfes 1 besteht üblicherweise aus einem Kunststoff wie Silikon oder PVC. Der Ankoppelbereich 4, vorzugsweise aber die gesamte Außenseite 5 des Koppelbalges sowie vorzugsweise auch dessen gesamte Innenseite 6 mit Nanodiamanten beschichtet, welche an ihrer Oberfläche Carboxylgruppen tragen. Die Carboxylgruppen tragenden Nanodiamanten (im folgenden wird aus Vereinfachungsgründen nur von Nanodiamanten gesprochen) werden mit Hilfe der dip-coating-Technik aufgebracht. Dazu wird der Koppelbalg in eine wässrige Suspension mit einer Diamantkonzentration von 0,5g/l eingetaucht und nach einer Ruhephase von 1 Stunde aus der Suspension heraus gezogen und getrocknet. Vor der Beschichtung wird der Koppelbalg 3 einen dreistufigen Reinigungsverfahren im Ultraschallbad für jeweils 20 min pro Stufe unterzogen. In der ersten Stufe wird mit deionisiertem Wasser, in der zweiten Stufe zur Entfettung mit Ethanol und in der dritten Stufe wiederum mit deionisiertem Wasser gereinigt

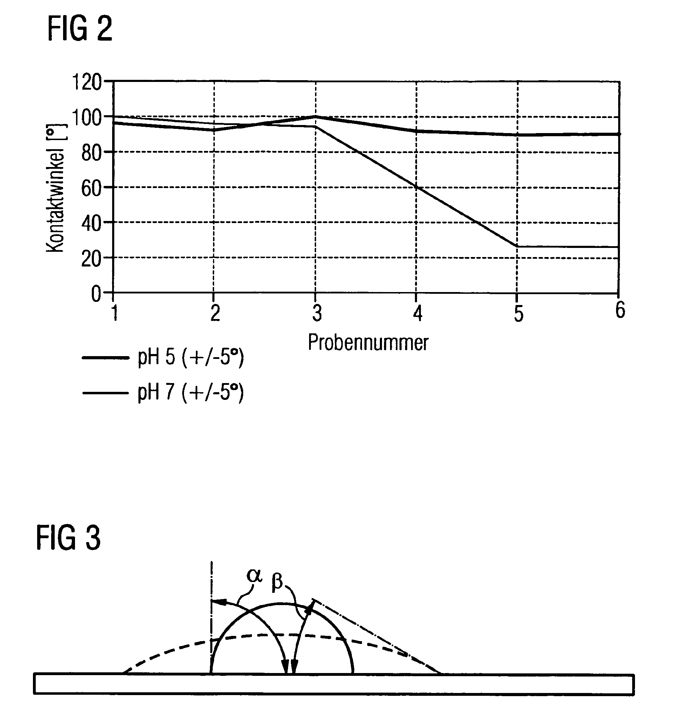

Um die oben bereits erwähnte Abhängigkeit der Qualität der Beschichtungen vom pH-Wert der Partikelsuspensionen zu testen, wurden Zuschnitte eines Koppelbalges aus Silikon mit Suspensionen unterschiedlichen pH-Werts behandelt. Wie der unten stehenden Tabelle zu entnehmen ist, wurden jeweils 6 verschiedenen Proben untersucht, wobei Probe 1 unbeschichtetes Balgmaterial ist und bei den Proben 2 bis 6 der pH-Wert der Partikelsuspension beginnend bei pH 5 bis pH 9 gesteigert wurde. Die pH-Wert Einstellung erfolgt mit Puffersubstanzen, z.B. mit einem Phosphatpuffer. Nach der Trocknung wurden die einzelnen Proben hinsichtlich ihres Verhaltens zu Lösungen unterschiedlichen pH-Wertes untersucht. Dazu wurde jeweils ein 20µl Tropfen auf die jeweilige Probe aufgebracht und durch fotographische Auswertung der Kontaktwinkel bestimmt. Der Kontaktwinkel wird an der Grenze zwischen Oberfläche und Tropfen gemessen und ist ein Maß für die Benetzbarkeit einer Oberfläche. Wie in Fig. 3 schematisch gezeigt ist, bildet sich bei einer relativ hydrophoben Oberfläche ein beispielsweise halb kugelförmiger Tropfen mit einem Kontaktwinkel α von etwa 90° aus. Je hydrophober die Oberfläche ist, desto geringer sind die Anziehungskräfte zwischen den Wassermolekülen und der Oberfläche, so dass die Adhäsionskräfte zwischen den Wassermolekülen überwiegen und es zur Bildung eines kugeligen Wassertropfens kommt. Ist dagegen die Oberfläche hydrophil, wird sie von dem Wasser mehr oder weniger vollständig benetzt, so dass sich beispielsweise ein flacher, in Fig. 3 mit gestrichelter Linie dargestellter Tropfen mit einem wesentlich kleineren Kontaktwinkel β von beispielsweise 35° ausbildet. Bei stark hydrophilen Oberflächen geht der Kontaktwinkel gegen 0°.

| Probe Nr. | PH der zur Beschichtung verwendeten Suspension |
|---|---|
| 1 | - |
| 2 | pH 5 |
| 3 | pH 6 |
| 4 | pH 7 |
| 5 | pH 8 |
| 6 | pH 9 |

Wird auf die unterschiedlichen Proben 1 bis 6 ein Tropfen neutralen Wassers (pH = 7) aufgebracht, so zeigt sich bei den Proben 2 und 3, bei denen die Suspensionen einen pH-Wert von 5 bzw. 6 aufwies, ein hoher Kontaktwinkel im Bereich von 100°. Die Proben 2 und 3 weisen somit eine Oberfläche auf, die hydrophob ist und im Wesentlichen jener der unbehandelten Probe 1 entspricht. Ein deutlich unterschiedliches Verhalten zeigt sich bei den Proben Nr. 4 - Nr. 6. Hier werden mit zunehmenden pH-Wert zunehmend kleinere Kontaktwinkel erreicht, d.h. die mit Nanodiamanten beschichtete Balgoberfläche wird zunehmend hydrophiler, wobei sich bei den Proben 5 und 6 praktisch kein Unterschied mehr zeigt. Dies bedeutet, dass sich die Qualität bzw. die Hydrophilie der Beschichtung nicht weiter steigern lässt, wenn in der Partikelsuspension ein über pH 8 hinausgehender pH-Wert eingestellt wird. Ein entsprechend den Probennummern 4, 5 oder 6 behandelter Koppelbalg weist somit eine ausreichende Benetzbarkeit seiner Oberfläche auf, so dass die eingangs erwähnten Problemen hinsichtlich Lufteinschlüsse und Kavitation vermieden oder zumindest soweit zurückgedrängt sind, dass sie nicht mehr stören.

Das Umstellen der Balgoberfläche von Hydrophil auf Hydrophob ist dem Diagramm gem. Fig. 2 ebenfalls entnehmbar. Hierzu wird ein 20µl Tropfen mit einem pH-Wert von 5 aufgebracht. Zur pH-Werteinstellung wurde ein Phosphatpuffer verwendet. Fig. 2 ist nun deutlich entnehmbar, dass bei den Proben 4, 5 und 6 ein Umschalten von hydrophil auf hydrophob erfolgt, was sich daran zeigt, dass die entsprechenden Kontaktwinkel im Bereich von etwa 20° bis 40° liegen. Bei der unbeschichteten Probe 1 und den nicht ausreichend beschichteten Proben 2 und 3, die ohnehin schon hydrophobe Oberflächen aufweisen, zeigt sich beim Aufbringen eines einem pH-Wert von 5 aufweisenden Wassertropfens kein Effekt.

Aus den oben stehenden Ausführungen ergibt sich somit, dass bei der Durchführung einer ESWL als Koppelfluid ein solches zu verwenden ist, welches basisch bis neutral (pH 7) eingestellt ist. Die Koppelbalgaußenseite 5 ist dann hydrophil und wird vom Koppelfluid optimal benetzt. Da eine gute Benetzung der Balgoberfläche auch an dessen Innenseite erforderlich ist, gilt für das im Koppelbalg enthaltene Übertragungsfluid hinsichtlich des pH-Wertes das oben Gesagte analog.

Nach der Durchführung einer ESWL wird der Koppelbalg gereinigt. Dazu wird eine Reinigungslösung mit einem pH-Wert im Bereich von pH 5 verwendet. Eine solche Reinigungslösung ist nur leicht sauer und dadurch wenig aggressiv, wodurch sie für eine Reinigung durchführende Person unschädlich ist und weder das Material des Koppelbalges oder sonstiger Teile des Stoßwellenkopfes angreift.

## Patentansprüche

1. Stoßwellenkopf (1) für eine Vorrichtung zur Stoßwellenbehandlung, umfassend einen aus einem Polymer bestehenden Koppelbalg (3) mit einem zur Ankopplung an einen Patienten dienenden Ankoppelbereich (4), **dadurch gekennzeichnet, dass** an der Außenseite (5) des Ankoppelbereichs polare funktionelle Gruppen tragende Nanopartikel vorhanden sind.

2. Stoßwellenkopf nach Anspruch 1, bei dem die Nanopartikel kohlenstoffhaltige Partikeln sind.

3. Stoßwellenkopf nach Anspruch 2, bei dem die Nanopartikel Nanodiamanten sind.

4. Stoßwellenkopf nach Anspruch 1, 2 oder 3, bei dem an die Nanopartikel organische Gruppen gekoppelt sind.

5. Stoßwellenkopf nach einem der vorhergehenden Ansprüche, bei dem die Nanopartikel funktionelle Gruppen mit einer Säure- und/oder Basenfunktion tragen.

6. Stoßwellenkopf nach Anspruch 5, an dessen Ankoppelbereich (4) Nanopartikel mit Carboxylgruppen vorhanden sind.

7. Stoßwellenkopf nach einem der vorhergehenden Ansprüche, dessen Koppelbalg aus einem polaren Kunststoff besteht.

8. Stoßwellenkopf nach einem der vorhergehenden Ansprüche, bei dem die Nanopartikel als Oberflächenbeschichtung auf den Koppelbalg aufgebracht sind.

9. Stoßwellenkopf nach einem der vorhergehenden Ansprüche, bei dem an der gesamten Außenseite des Koppelbalgs polare funktionelle Gruppen tragende Nanopartikel vorhanden sind.

10. Stoßwellenkopf nach einem der vorhergehenden Ansprüche, bei dem an der Innenseite des Koppelbalgs, zumindest an jener des Ankoppelbereichs, polare funktionelle Gruppen tragende Nanopartikel vorhanden sind.

11. Verfahren zur Herstellung eines Stoßwellenkopfes nach einem der vorhergehenden Ansprüche, **dadurch gekennzeichnet, dass** auf die Außen- bzw. Innenseite des Koppelbalges eine wässrige Suspension der polare funktionelle Gruppen tragenden Nanopartikel aufgebracht und anschließend der Koppelbalg einer Trocknung unterzogen wird.

12. Verfahren nach Anspruch 11, bei dem eine Suspension verwendet wird, die Nanopartikel mit sauren- und/oder basischen funktionelle Gruppen enthält, wobei in der Suspension ein pH-Wert eingestellt wird, bei dem die Säure-Base-Reaktion zwischen den genannten Gruppen und dem Wasser der Suspension zumindest teilweise stattfindet.

13. Verfahren nach Anspruch 12, bei dem Nanopartikel mit Carboxylgruppen verwendet werden, wobei in der Suspension ein pH-Wert größer 6 eingestellt wird.

14. Verfahren nach einem der Ansprüche 11 bis 13, bei dem eine Suspension verwendet wird, die einen Gehalt an Nanopartikeln von 0,1 g/l bis 1,5 g/l aufweist.

15. Verfahren nach Anspruch 14, bei dem die Suspension einen Partikelgehalt von 0,3 g/l bis 0,7 g/l aufweist.

## Claims

1. Shock wave head (1) for a shock wave treatment device, comprising a coupling bellows (3) consisting of a polymer and having a coupling region (4) serving for coupling to a patient, **characterised in that** nanoparticles carrying polar functional groups are present on the outside surface (5) of the coupling region.

2. Shock wave head according to claim 1, wherein the nanoparticles are carbon-containing particles.

3. Shock wave head according to claim 2, wherein the nanoparticles are nanodiamonds.

4. Shock wave head according to claim 1, 2 or 3, wherein organic groups are coupled to the nanoparticles.

5. Shock wave head according to one of the preceding claims, wherein the nanoparticles carry functional groups having an acid and/or base function.

6. Shock wave head according to claim 5, at the coupling region (4) of which nanoparticles containing carboxyl groups are present.

7. Shock wave head according to one of the preceding claims, the coupling bellows of which consists of a polar plastic.

8. Shock wave head according to one of the preceding claims, wherein the nanoparticles are applied to the coupling bellows as a surface coating.

9. Shock wave head according to one of the preceding claims, wherein nanoparticles carrying polar functional groups are present on the entire outside surface of the coupling bellows.

10. Shock wave head according to one of the preceding claims, wherein nanoparticles carrying polar functional groups are present on the inside surface of the coupling bellows, at least on the inside surface of the coupling region.

11. Method for producing a shock wave head according to one of the preceding claims, **characterised in that** an aqueous suspension of the nanoparticles carrying polar functional groups is applied to the outside and/or inside surface of the coupling bellows and subsequently the coupling bellows is subjected to a drying process.

12. Method according to claim 11, wherein a suspension containing nanoparticles containing acidic and/or basic functional groups is used, with a pH value being set in the suspension in which the acid-base reaction takes place at least partially between the said groups and the water of the suspension.

13. Method according to claim 12, wherein nanoparticles containing carboxyl groups are used, with a pH value greater than 6 being set in the suspension.

14. Method according to one of claims 11 to 13, wherein a suspension is used having a nanoparticle content of 0.1 g/l to 1.5 g/l.

15. Method according to claim 14, wherein the suspension has a particle content of 0.3 g/l to 0.7 g/l.

## Revendications

1. Tête ( 1 ) d'onde de choc pour un dispositif de traitement par onde de choc, comprenant un soufflet ( 3 ) de couplage ayant une zone ( 4 ) de couplage servant au couplage avec un patient, **caractérisée en ce qu'**il y a des nanoparticules portant des groupes fonctionnels polaires sur le côté ( 5 ) extérieur de la zone de couplage.

2. Tête d'onde de choc suivant la revendication 1, dans laquelle les nanoparticules sont des particules contenant du carbone.

3. Tête d'onde de choc suivant la revendication 2, dans laquelle les nanoparticules sont des nanodiamants.

4. Tête d'onde de choc suivant la revendication 1, 2 ou 3, dans laquelle des groupes organiques sont couplés aux nanoparticules.

5. Tête d'onde de choc suivant l'une des revendications précédentes, dans laquelle les nanoparticules portent des groupes fonctionnels ayant une fonction acide et/ou une fonction basique.

6. Tête d'onde de choc suivant la revendication 5, sur la zone ( 4 ) de couplage de laquelle il y a des nanoparticules ayant des groupes carboxyles.

7. Tête d'onde de choc suivant l'une des revendications précédentes, dans laquelle le soufflet de couplage est en une matière plastique polaire.

8. Tête d'onde de choc suivant l'une des revendications précédentes, dans laquelle les nanoparticules sont déposées sous la forme d'un revêtement de surface sur le soufflet de couplage.

9. Tête d'onde de choc suivant l'une des revendications précédentes, dans laquelle il y a des nanoparticules portant des groupes fonctionnels polaires sur toute la surface extérieure du soufflet de couplage.

10. Tête d'onde de choc suivant l'une des revendications précédentes, dans laquelle il y a des nanoparticules portant des groupes fonctionnels polaires sur le côté intérieur du soufflet de couplage, au moins sur chaque zone de couplage.

11. Procédé de fabrication d'une tête d'onde de choc suivant l'une des revendications précédentes, **caractérisé en ce que** l'on dépose une suspension aqueuse des nanoparticules portant des groupes fonctionnels polaires sur le côté extérieur ou sur le côté intérieur du soufflet de couplage et on soumet ensuite le soufflet de couplage à un séchage.

12. Procédé suivant la revendication 11, dans lequel on utilise une suspension, qui contient des nanoparticules ayant des groupes fonctionnels acides et/ou basiques, en établissant dans la suspension un pH, auquel la réaction acide-base entre lesdits groupes et l'eau de la suspension a lieu, au moins en partie.

13. Procédé suivant la revendication 12, dans lequel on utilise des nanoparticules ayant des groupes carboxyles, en établissant un pH supérieur à 6 dans la suspension.

14. Procédé suivant l'une des revendications 11 à 13, dans lequel on utilise une suspension, qui a une teneur en nanoparticules de 0, 1 g/l à 1,5 g/l.

15. Procédé suivant la revendication 14, dans lequel la suspension a une teneur en particules de 0,3 g/l à 0,7 g/l.
